(19) Europäisches Patentamt
European Patent Office
Office européen des brevets

(11) Publication number: **0 139 481 B1**

(12) # EUROPEAN PATENT SPECIFICATION

(45) Date of publication of patent specification: **13.05.92**

(51) Int. Cl.[5]: **A61K 7/021**, C09C 3/08

(21) Application number: **84306449.4**

(22) Date of filing: **21.09.84**

The file contains technical information submitted after the application was filed and not included in this specification

(54) **Surface modification using N-acyl lysines.**

(30) Priority: **22.09.83 JP 175708/83**
**22.09.83 JP 175709/83**
**19.06.84 JP 126257/84**
**26.06.84 JP 131781/84**

(43) Date of publication of application:
**02.05.85 Bulletin 85/18**

(45) Publication of the grant of the patent:
**13.05.92 Bulletin 92/20**

(84) Designated Contracting States:
**DE FR GB**

(56) References cited:
**FR-M- 6 475**
**US-A- 3 897 466**
**US-A- 4 016 287**
**US-A- 4 606 914**

(73) Proprietor: **AJINOMOTO CO., INC.**
**5-8, Kyobashi 1-chome, Chuo-ku**
**Tokyo 104(JP)**

(72) Inventor: **Meguro, Kenjiro**
**3-26-3 Nakano Nakano-ku**
**Tokyo(JP)**
Inventor: **Sagawa, Kiochiro**
**2-20-8 Kannon Kawasaki-ku**
**Kawasaki-shi Kanagawa-ken(JP)**
Inventor: **Yokota, Hirofumi**
**958 Kashimada Saiwai-ku**
**Kawasaki-shi Kanagawa-ken(JP)**
Inventor: **Takehara, Masahiro**
**3-3-9 Kataseyama**
**Fujisawa-shi Kanagawa-ken(JP)**

(74) Representative: **Bond, Bentley George et al**
**Haseltine Lake & Co. 28 Southampton Buildings Chancery Lane**
**London WC2A 1AT(GB)**

**EP 0 139 481 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the use of N-acyl lysines as surface modifiers.

The preparation of N-acyl derivatives of basic amino acids, e.g. lysine, is described in US-A-3897466.

US-A-4606914 describes a cosmetic composition comprising an N-acylamino acid, wherein the acyl group includes a residue of capric acid, lauric acid, myristic acid, palmitic acid or stearic acid.

According to the present invention, there is provided a substance whose surface has been modified with one or more N-mono- or N,N'-di-acylated lysines, wherein the acyl group in the N-mono-acylated lysine is an aliphatic acyl group having from 8 to 22 carbon atoms, and each of the acyl groups, which may be the same or different, in the N,N'-di-acylated lysine is an aliphatic acyl group having from 1 to 22 carbon atoms with the proviso that the two aliphatic acyl groups together have from 9 to 44 carbon atoms.

According to a first preferred aspect of the present invention, the acylated lysine is one represented by the following general formulae:

$$RCONH(CH_2)_4-\underset{\displaystyle NH_2}{\underset{|}{CH}}-COOH \qquad (I)$$

$$H_2N(CH_2)_4-\underset{\displaystyle NHCOR}{\underset{|}{CH}}-COOH \qquad (2)$$

$$R'CONH(CH_2)_4-\underset{\displaystyle NHCOR''}{\underset{|}{CH}}-COOH \qquad (3)$$

(wherein RCO in general formulae (1) and (2) stands for an aliphatic acyl group of 8 to 22 carbon atoms and R'CO and R''CO in general formula (3) each stand for an aliphatic acyl group of 1 to 22 carbon atoms, providing that the two aliphatic acyl groups jointly have 9 to 44 carbon atoms).

In recent years, in various fields such as of paints, resins, and electronics, composite materials prepared by mixing and dispersing such inorganic fillers as calcium carbonate, titanium dioxide, talc, silica, ferrite, metallic fibers, and glass fibers in such organic media as resins and paints have come to be used for the purpose of imparting strength and toughness, enhancing resistance to heat and weather conditions, improving color tone and viscosity, reducing cost by adulteration, and conferring electroconductivity as a newly acquired property.

Generally these inorganic fillers have their surfaces covered with polar groups such as hydroxyl groups and adsorbed moisture and, therefore, are deficient in affinity for oil. In their unaltered form, they cannot be uniformly dispersed in organic media such as paints and resins without difficulty. Thus, they fail to produce composite materials as aimed at.

In actuality, various surface modifiers are utilized for the purpose of improving wettability and enhancing dispersibility of such fillers. Examples of such surface active agents are dodecyl sulfuric ester salts, alkylbenzene sulfonates, fatty acid salts, dialkyl sulfosuccinic ester salts, polyoxyethylene alkyl ethers, polyoxyethylene sorbitan fatty esters, quaternary ammonium salts, lecithin, and alkyl betaine, polymers such as polystyrene, polypropylene, polyesters, styrene-methacrylic acid type copolymers, and styrene-acrylic acid type copolymers, and silane type coupling agents.

These inorganic surface modifiers are required to possess characteristic properties, i.e. (1) the ability to manifest a high dispersing effect at a low application rate, (2) the ability to impart a strong adsorbing property to the surface of the filler, to avoid easy separation from treated boundary region, and (3) extensive adaptability to various fillers.

In the aforementioned inorganic surface modifiers which have found popular acceptance to date, such surface active agents as stearates, for example, are known to suffer from the disadvantages that they produce insufficient modifying effects at low application rates and they induce the phenomenon of bluing during the processing of resins and impair water repellency and weatherability of end products at excessively high application rates. When the surface modification is tried with polymers such as polystyrene, there is the possibility that, depending on the kind of organic solvent used as a dispersant, the polymer coat formed on the surface of the inorganic filler will swell or dissolve and separate from the surface.

When silane type coupling agents are used, they manifest an excellent effect of modification on silicon-containing inorganic substances such as glass and silica because the molecules of silane type coupling agents react with their surface functional groups and form strong adsorbent films. They, however, fail to manifest the expected effect upon other inorganic fillers such as aluminium trihydrate and manifest absolutely no effect upon calcium carbonate. Thus, this lack of indiscrimination in the selection of fillers by kind is a drawback for such silane type coupling agents.

In the circumstance, the inventors continued a diligent study with a view to developing a novel surface modifier satisfying the aforementioned characteristic properties that the surface modifiers for inorganic substances are expected to fulfil. They have, consequently, found that the acylation product of lysine which is one type of amino acid manifests an outstanding surface modifying effect at a low application rate, avoids inducing separation from the surface of adsorption in various organic solvents, enjoys extensive adaptability to fillers, and serves as an outstanding surface modifier for inorganic substances. This knowledge has led to the perfection of this aspect of the invention.

The $N^{\alpha}$ - $N^{\epsilon}$ -diacyl lysine represented by the aforementioned general formula may be obtained by the so-called Schotten Baumann reaction, i.e. the instillation of fatty acid chloride into an alkaline aqueous solution of lysine. In this case, the compounds (1) and (2), which have the $\epsilon$-position or $\alpha$-position selectively acrylated, are obtained, by ensuring that the amino groups at the $\alpha$-position or $\epsilon$-position protected in advance. The compound (1) can also be produced by thermally dehydrating a fatty acid of lysine at a temperature in the range of 100° to 250°C (Japanese Patent Publication SHO 51 (1976)-28610).

Examples of the N-acyl lysine to be used in this aspect of the invention include $N^{\alpha}$-octanoyl lysine, $N^{\alpha}$-oleoyl lysine, $N^{\alpha}$-lauroyl lysine, $N^{\alpha}$-myristoyl lysine, $N^{\alpha}$-palmitoyl lysine, $N^{\alpha}$-stearoyl lysine, $N^{\epsilon}$-octanoyl lysine, $N^{\epsilon}$- oleoyl lysine, $N^{\epsilon}$-lauroyl lysine, $N^{\epsilon}$-myristoyl lysine, $N^{\epsilon}$-palmitoyl lysine, $N^{\epsilon}$-stearoyl lysine, $N^{\alpha}$-lauroyl-$N^{\epsilon}$-stearoyl lysine, $N^{\alpha}$-hexanoyl-$N^{\epsilon}$-palmitoyl lysine, and $N^{\alpha}$-$N^{\epsilon}$-dilauroyl lysine, and their mixtures. This aspect of the invention does not discriminate these N-acyl lysines by the question as to whether they are in optical active form or in racemic form.

It is known that $N^{\epsilon}$-acyl lysines or metal salts thereof serve as stabilizers for halogen-containing resins such as, for example, polyvinyl chloride (Japanese Patent Application Laid-open SHO 51 (1976)-12847). Nothing has been disclosed about their utility as surface modifiers for inorganic substances. This particular utility has been brought to light for the first time by the inventors.

The surface modification by the use of N-acyl lysine can be effected by (1) the method which comprises adding the modifier in its unaltered form to the filler to be modified and communuting the resultant mixture in a grinder such as a ball mill, an atomizer, or a colloid mill, (2) the method which comprises stirring the modifier in conjunction with the filler in a suitable organic solvent and then treating the resultant solution to expel the solvent, and (3) the method which comprises directly adding the modifier to a mixture of an organic medium with the filler and blending the resultant mixture such as with hot rolls. The method (2) proves advantageous over the other methods in the sense that it produces the desired surface modification quite effectively at an extremely low application rate. When the surface modification is tried on calcium carbonate by following this particular method, for example, ample surface modification is effectively obtained at a minimal application rate of 0.05% based on the amount of calcium carbonate under treatment.

The N-acyl lysine employed in this aspect of the present invention can be used for a very wide variety of inorganic fillers such as calcium carbonate, titanium dioxide, kaolin, talc, silica, ferrite, chromium dioxide, metallic fibers, glass fibers, and asbestos. The aforementioned inorganic fillers which have been given a surface modification by the use of the N-acyl lysine can be effectively mixed in various organic media such as polyurethane resin, vinyl chloride-vinyl acetate copolymer, epoxy resin, phenol resin, acryl resin, cellulose resin, polyamide resin, and urea resin.

When inorganic pigments treated by the conventional method of surface modification are used in paints, it is frequently observed that the modifier adsorbed on the surface of the inorganic pigments dissolves out into the organic solvent to induce coagulation and separation of the inorganic pigment and impair smooth surface and even color of the produced coats. In contrast, the inorganic pigments treated by the use of N-acyl lysine of the present invention have the advantage that they induce coagulation and separation only sparingly in various organic solvents including ketones such as methylethyl ketone, methylisobutyl ketone, and cyclohexanone, esters such as ethyl acetate, and hydrocarbons such as toluene, and rarely impair the quality of paints.

As described above, the N-acyl lysines possess excellent properties as surface modifier. These properties are fully manifested not merely when the N-acyl lysine is used by itself but also when it is used in combination with other surface improvers.

According to a second preferred aspect of the present invention, there is provided a formulation having compounded therein at least one N-acyl-lysine represented by general formulae (1) to (3) below:

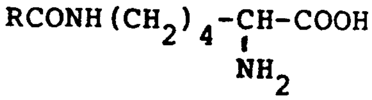

(1)

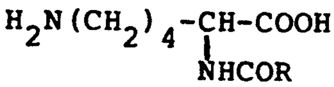

(2)

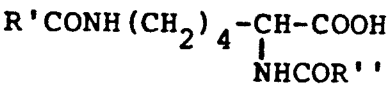

(3)

wherein RCO in general formulae (1) and (2) represents an aliphatic acyl group having 8 to 22 carbon atoms; and R'CO and R''CO in general formula (3) represent an aliphatic acyl group having 1 to 22 carbon atoms, provided that the total number of both carbon atoms is 9 to 44. The formulations of this aspect of the invention are useful as cosmetic compositions.

The second preferred aspect of the present invention relates to cosmetic compositions having formulated therein N-acyl-lysines in a powdery form.

As raw materials for cosmetic powders, there have been heretofore known inorganic powders such as titanium dioxide, zinc powder, talc, kaolin, precipitated calcium carbonate, basic magnesium carbonate, etc., or organic powders comprising synthetic high molecular weight substances such as nylon 12, cross-linked polystyrene, etc. For these cosmetic powders, properties that (1) the surface of the skin be masked to veil spots and freckles, (2) the spreading and smoothness in applying to the skin be excellent, (3) the adherence to the skin be excellent, (4) the absorptivity of sweat and secretion be good, (5) they be easily compatible with other base materials for cosmetics, (6) their safety be high, etc. are required.

However, these inorganic powders have the disadvantages that titanium dioxide, etc. have high hydrophilic properties and are only difficultly compounded with oily base materials, although they have a good masking power and a good feel in use; further, while the pH of the skin is between 5 and 6, talc has a markedly high pH and a strong surface activity and therefore, has serious restrictions in actual use from the viewpoint of safety.

On the other hand, in organic powders such as nylon 12, cross-linked polystyrene, etc. which have been recently utilized as novel materials in the field of fragrances and cosmetics, they are advantageous in having a good smoothness resulting from their fine, spherical form and high safety but, on the other hand, the disadvantage that makeup tends to easily come off due to poor dispersibility in aqueous base materials, poor absorbability of sweat, secretions, etc. and poor adsorption to the skin.

In recent years, various attempts to improve the aforesaid disadvantages have been made by subjecting these powders to an appropriate surface treatment.

These attempts are, for example, a method for improving dispersibility in oily base materials and safety which comprises subjecting titanium dioxide to surface treatment with mono-esters of long chain fatty acids and higher alcohols to render the surface hydrophobic (Japanese Published Unexamined Patent Application 124627/78), a method for improving the masking power and adsorption to the skin which comprises coating the surface of talc with polyamino acids to thereby relieve irritation (Japanese Published Unexamined Patent Application 79315/80), a method for improving dispersibility and adsorption of aqueous stains which comprises coating the surface of nylon 12 powders with titanium dioxide (COSMETICS AND TOILETRIES, 94(12), 33 (1979) ), etc.

Even though these surface treatments, namely, the method for rendering hydrophilic powders hydrophobic or the method for rendering hydrophobic powders hydrophilic, are used, however, nothing has been found to sufficiently meet the aforesaid properties required for cosmetic powders. In addition, the actual circumstances newly encounter problems that costs increase accompanied by the surface treatment, operability becomes poor and the production of powders having a constant quality is difficult.

As a result of extensive investigations on novel powdery materials utilizable as raw materials for cosmetics, the present inventors have found that powders of N-acyl-lysines described above are difficultly soluble in various solvents such as water, squalane, liquid paraffin, etc., specifically among various acyl-amino acids, do not swell and maintain a stable form as powders even in these solvents, are mild to the skin and, when compounded as a component in cosmetic raw materials, cosmetics having excellent adherence to the skin and absorbability of secretions can be produced and, have accomplished the second aspect of the present invention.

That is, the second aspect of the present invention relates to cosmetic compositions having compounded therein at least one of N-acyl-lysines represented by general formulae (1) to (3) given above, in a powdery form.

In order to obtain the $N^{\alpha}$-$N^{\epsilon}$-diacyl-lysines shown by general formula (3) described above, a reaction which comprises dropwise adding fatty acid chlorides in an alkaline aqueous solution of lysine, the so-called Schotten-Baumann reaction may be used. In this case, when the amino group at the $\alpha$-position or $\epsilon$-position is previously protected, Compounds (1) and (2) wherein the $\epsilon$-position or $\alpha$-position is selectively acylated can be obtained. Further, Compound (1) may also be obtained by heating and dehydrating fatty acid salts of lysine at temperatures of 100 to 250°C (Japanese Published Examined Patent Application 28610/76). The resulting N-acyl-lysines may be removed by solid-liquid separation such as filtration, etc. After decolorising and purifying, if necessary, the N-acyl-lysines may be pulverised to the desired graininess using grinders such as a ball mill, a colloid mill, an atomizer, etc. to obtain the desired powders.

As acyl groups, aliphatic acyl groups having 8 to 22 carbon atoms are generally used. However, it is possible to control the hydrophilic properties hydrophobic properties, smoothness, adherence, masking power, etc. of N-acyl-lysines by modifying the number of carbon atoms and the number of acyl groups.

Examples of the N-acyl-lysines used in this aspect of the present invention include $N^{\alpha}$-octanoyl-lysine, $N^{\alpha}$-oleoyl-lysine, $N^{\alpha}$-lauroyl-lysine, $N^{\alpha}$-myristoyl -lysine, $N^{\alpha}$-palmityol-lysine, $N^{\alpha}$-stearoyl-lysine, $N^{\epsilon}$-octanoyl-lysine, $N^{\epsilon}$-oleoyl-lysine, $N^{\epsilon}$-lauroyl-lysine, $N^{\epsilon}$-myristoyl-lysine, $N^{\epsilon}$-palmitoyl-lysine, $N^{\epsilon}$-stearoyl-lysine, $N^{\alpha}$-lauroyl-$N^{\epsilon}$-stearoyl-lysine, $N^{\alpha}$-hexanoyl-$N^{\epsilon}$ -palmitoyl-lysine, $N^{\alpha}$-$N^{\epsilon}$-dilauroyl-lysine, etc. and mixtures thereof. These N-acyl-lysines may be employed even though they may be optically active or in a racemic form.

It is known that N -acyl-lysines or metal salts thereof can be used as stabilisers for halogen-containing resins such as polyvinyl chloride, etc. (Japanese Published Unexamined Patent Application 12847/76). However, there is no disclosure in relation to their use as cosmetic powders, which has been first found by the present inventors.

As is evident from the structure, the N-acyl-lysines used in the present invention are composed of natural lysine and fatty acids and, are less irritative to the skin and show excellent adsorpability and masking power because they contain amido groups similarly to skin proteins. In addition, the N-acyl-lysines possess hydrocarbon groups which are hydrophobic groups and amino groups and carboxyl groups which are hydrophilic groups, in the molecule thereof. The balance of the hydrophilic groups and the hydrophobic groups can easily be changed by modifying the number and chain length of acyl groups to be introduced. Accordingly, the dispersibility in oily base materials or aqueous base materials and the absorpability of sweat, secretions, etc. can be controlled, whereby it is possible to choose a particular N-acyl-lysine depending upon physical properties of the desired cosmetics. Thus, the N-acyl-lysines are epoch-making powders for cosmetics. Such powders may be used singly. Further, even when they are used in combination with other powders for cosmetics, the aforesaid characteristics are satisfactorily exhibited.

Cosmetics indended in the present invention include face powders, paste powders, solid powders, body powders, baby powders, antihidrotics, foundations, tooth pastes, etc. In addition thereto, the cosmetics may also include those containing powders therein.

According to a third preferred aspect of the present invention there is provided a method for the modification of the surface of an inorganic substance, characterized by causing said surface to adsorb a salt of lysine with a fatty acid of 8 to 22 carbon atoms and subsequently heating the substance at a temperature in the range of 100° to 250° C.

This third aspect of the present invention aims to obtain inorganic substances with surfaces modified so as to be adaptable for a wide variety of applications such as in paints, resins, medicines, cosmetics, and paper products.

Heretofore, bentonite, talc, kaolin , diatomaceous earth, light-weight silicic anhydride, mica, calcium secondary phosphate, silica, alumina, barium sulphate, titanium dioxide, zinc oxide, iron oxide yellow, iron oxide red, iron oxide black, ultramarine, chromium hydroxide, chromium oxide, montmorillonite, calcium carbonate, ferrite, metallic powder, metallic fibres, glass fibres, and asbestos have been used for the purpose of imparting strength and toughness, improving thermal resistance, improving colour tone and

viscosity, decreasing cost by adulteration, and conferring electroconductivity, etc. as acquired quality in the field of paints and resins and as principal bases for excipients, pigments, makeup articles in the field of medicines and cosmetics.

The surfaces of such inorganic substances are generally covered with polar groups such as hydroxyl groups and adsorped moisture and, therefore, are deficient in affinity for oils and liable to be wetted with water. Thus, these inorganic substances cannot be uniformly dispersed in organic media such as paints and resins without difficulty. When such inorganic substances are used as bases for makeup cosmetic articles, they are wetted with perspiration so readily as to cause dissolution of the applied coats of makeup. This is a notorious drawback for inorganic substances. It is also known that such inorganic substances as described above have on their surfaces places exhibiting acidity and places exhibiting basicity and, because of their high surface activity, irritate the skin, accelerate degradation and decomposition of coexisting medicines or perfumes, or entail other similar problems.

In the circumstance, various methods of surface modification have been proposed for the purpose of improving the wettability, enhancing the dispersibility, repressing the surface activity, curbing the skin-irritating property of organic substances, and promoting the stability of coexisting medicines and perfumes.

Methods of surface treatment by the use of surface active agents such as dodecyl sulfates, alkylbenzene sulfonates, fatty acid salts, dialkylsulfosuccinates, polyoxyethylene alkyl ethers, polyoxyethylene sorbitan fatty acid esters, glycerin fatty acid esters, quaternary ammonium salts, lecithin, and alkyl betaine, methods of surface treatment by the use of polymers such as polystyrene, polypropylene, polyesters, styrene-methacrylic acid type copolymers, styrene-acrylic acid type copolymers, polytetrafluoroethylene, polychlorotrifluroethylene, polyethylene-tetrafluoroethylene type copolymers, polyaspartic acid, polyglutamic acid, and polyglumatic acid-methyl esters, and methods of surface treatment by the use of silane compounds and alcohol compounds are examples.

Surface active agents such as zinc stearate which are used in some of the methods of surface modification of inorganic substances described above are widely used in the field of paints and resins. It is known that when they are used at low application rates, they are not sufficiently effective in improving the dispersibility and, when they are used at excessively high rates, they have the disadvantage of causing the phenomenon of bluing during the processing of the resins and of degrading the water-repellency and weatherability of the end products. It is known in the art to treat inorganic powders for cosmetics with surface active agents such as metallic soap and lecithin so as to modify the surface of the powders and to preclude the possible dissolution of cosmetic makeups. These surface active agents, however, manifest virtually no effect in repressing the activity of inorganic surfaces and fail to solve the problem of degradation of perfumes and coloring matter. They are inevitably required, therefore, to be used in combination with antioxidants or chelating agents, for example.

When the surface modification is tried with polymers such as polystyrene, there is the possibility that, depending on the kind of organic solvent serving as a dispersant, the polymer coat formed on the surface of the inorganic substance will swell or dissolve and separate from the surface. When the inorganic substance with such a polymer coat is used in paints, therefore, there ensues the inconvenience that this inorganic substance discriminates against the solvent by its kind. Further, when the inorganic powder which has undergone the surface modification by the use of such a polymer is used in cosmetics, since the surface is no longer hydrophilic, the possibility of the applied coat of makeup being wetted with and dissolved by perspiration is eliminated. Nevertheless, the residual monomer in the used polymer has the possibility of manifesting toxicity. Besides, during the treatment with the polymer for surface modification, the treatment inevitably entails use of a highly toxic solvent such as dichloroethane. These points pose as new problems in the face of this particular field which urges safety as the most exacting requirement. In case that silane compounds are used, these compounds manifest an excellent effect of modification on silicone-containing inorganic substances such as glass and silica because they react with their surface functional groups and form strong adsorbent films. They, however, have a poor effect upon other inorganic substances such as aluminium trihydrate and manifest absolutely no effect upon calcium carbonate which is widely used as an inorganic filler. Thus, this lack of indiscrimination in the selection of inorganic substances by kind is one drawback of these silane compounds.

Although various methods have been developed for surface modification of inorganic substances as described above, none of them have been able to produce inorganic substances which excel in dispersibility in organic solvents, resistance to water and solvents, and indiscrimination in the selection of inorganic substances by kind, and which enjoy repressed surface activity.

The inventors continued a diligent study with a view to overcoming such an unsatisfactory state of affairs. They have consequently found that modified inorganic substances satisfying all the requirements enumerated above are obtained by a method comprising the steps of causing the surface of a given

inorganic substance to adsorb a salt of lysine with a fatty acid of 8 to 22 carbon atoms and subsequently heating the substance at a temperature in the range of 100° to 250°C. This knowledge has led to the perfection of this aspect of the invention.

This aspect of the invention does not discriminate the N-acyl lysines by the question whether they are in optically active form or in racemic form.

For use in this invention, the fatty acid is required to have from 8 to 22 carbon atoms. Examples of the fatty acid satisfying this requirement include caproic acid, capric acid, lauric acid, myristic acid, palmitic acid, stearic acid, oleic acid, behenic acid, iso-stearic acid, palm oil fatty acid, and hard beef tallow fatty acid.

The characteristic of this aspect of the invention resides in causing the surface of an inorganic substance to adsorb a fatty acid salt of lysine and thereafter heating the inorganic substance at a temperature in the range of 100° to 250°C. This treatment includes a reaction of dehydrating amination and converts the fatty acid salt of the lysine into an N-acyl lysine which excels in resistance to water and solvents and, as the result, effects the surface effect aimed at. If the heating is effected at a lower temperature than specified, the conversion of the fatty acid salt of the lysine to the N-acyl lysine does not sufficiently proceed. The modified inorganic substance obtained consequently, though improved in dispersibility in organic solvents, possesses poor resistance to water and organic solvents, suffers from loss of weatherability, and causes dissolution of the applied coats of makeup by perpiration. If, on the other hand, the heating is carried out at a temperature exceeding 250°C, the fatty acid or the lysine undergoes oxidative decomposition and, as the result, the effect of surface modification does not reached the expected level and the treated inorganic substance takes up color and odor. The temperature of this heating must be maintained in the range of 100° to 250°C, preferably in the range of 130° to 180°C.

Although the time required for the heating treatment is variable with the temperature used and the quantity of the inorganic substance under treatment it is usually in the range of 30 minutes to 5 hours, preferably two to three hours where the treatment is carried out at a temperature in the range of 130°C to to 180°C. In this case, for the purpose of precluding the oxidative decomposition of the reactants, the heating treatment may be carried out under a vacuum or in an atmosphere of nitrogen gas.

The adsorption of the fatty acid salt of the lysine by the surface of the inorganic substance may be affected by (1) the method which comprises adding the fatty acid salt of the lysine to the inorganic substance to be modified and comminuting the resulting mixture in a grinder such as a ball mill, an atomizer, or a colloid mill thereby mechanochemically inducing the adsorption or (2) the method which comprises adding the inorganic substance to a solution of the fatty acid salt of the lysine, stirring the resultant mixture, and thereafter removing the solvent either by filtration or by heating under a vacuum. The method (2) proves to be advantageous over method (1) in that it permits uniform surface adsorption even when the organic substance under treatment is present in a small amount.

The amount of the fatty acid salt of lysine to be used in the surface treatment is desirably in the range of 0.1 to 10% by weight, preferably 0.2 to 2% by weight, based on the amount of the inorganic substance to be modified. If the amount is less than the lower limit of the range specified, the treatment does not give ample effect of modification without difficulty. If the amount exceeds the upper limit of the range, the treatment proves to be uneconomical because the excess of the amount added is only wasted.

Compared with the inorganic substances modified by the use of N-acyl lysine as contemplated by the first preferred aspect of the invention, the inorganic substances obtained by the surface modification described above enjoys the advantage that the cost of treatment is notably low and that since the fatty acid salt of lysine is soluble in alcohols, the treatment can be easily carried out in a wet condition to produce a uniformly modified surface.

The method of surface modification by this invention can be applied extensively to various inorganic substances.

Examples of such inorganic substances include bentonite, talc, kaolin, diatomaceous earth, lightweight silicic anhydride, mica, calcium secondary phosphate, silica, alumina, barium sulfate, titanium dioxide, zinc oxide, iron oxide yellow, iron oxide red, iron oxide black, ultramarine, chromium hydroxide, chromium oxide, montmorillonite, calcium carbonate, ferrite, metallic powder, metallic fibers, glass fibers, and asbestos.

The inorganic substances which have been given a surface modification by the method of this aspect of the invention can be easily dispersed in organic media such as polyurethane resin, vinyl chloride-vinyl acetate copolymer, epoxy resin, phenol resin, acryl resin, polyamide resin, cellulose resin, and urea resin. When the inorganic pigments treated by the conventional method of surface modification are used as in paints, it is frequently observed that the modifier adsorbed on the surface of the inorganic pigments dissolves out into the organic solvent to induce coagulation and separation of inorganic pigments and impair smooth surface and even color of the produced coats. In contrast, the inorganic pigments of modified

surface obtained by the method of this aspect of the invention have the advantage that they are excellent in resistance to solvents, induce coagulation and separation only sparingly in various organic solvents including ketones such as methylethyl ketone, methylisobutyl ketone, and cyclohexanone, esters such as ethyl acetate, and hydrocarbons such as toluene and normal paraffin, and rarely impair the quality of paints.

The inorganic substances of modified surface obtained by the method of this aspect of the invention possess improved water repellency and adhesiveness to the skin and sparingly cause solution of applied coats of makeup. Moreover, they enjoy smooth feel and notably repressed surface activity as compared with the inorganic substances not treated by the method of this aspect of the invention. Thus, they manifest outstanding quality when used in cosmetics. As described above, the inorganic substances which have been given surface modification by the method of this aspect of the invention can be applied to the commercial production of paints and resins and to the manufacture of medicines and cosmetics.

According to a fourth preferred aspect of the invention, an inorganic pigment is surface-treated with an N-mono-acylated lysine, with the acyl group having a long chain containing an aliphatic acyl group having 8 to 22 carbon atoms therein. Preferably, said N-mono-acylated basic amino acid with acyl having a long chain is selected from an $N^{\alpha}$ -long chain acyl-lysine and an $N^{\epsilon}$-long chain acyl-lysine. The formulations of this aspect of the present invention are useful as cosmetic compositions.

This aspect of the present invention relates to improved cosmetic compositions having formulated therein inorganic pigments. In cosmetic compositions, talc, kaolin , titanium dioxide, zinc flower, iron oxides, ultramarine and other inorganic pigments have been used heretofore. For cosmetics using such inorganic pigments, properties that (1) the surface of the skin be masked to veil spots and freckles, (2) spreading and smoothness in applying to the skin be excellent, (3) adherence to the skin be excellent, (4) the absorptivity of sweat and secretion to be good, (5) components be homogenously compatible to give smooth texture, (6) safety to be high, etc. are required.

However, conventional inorganic pigments are poor in compatibility with and dispersibility in oily bases because the surfaces thereof are hydrophilic so that homogenous cosmetics having smooth texture are obtained only with difficulty. In addition, when the inorganic pigments are spread over the skin, they have the disadvantage that the adherence to the skin is insufficient and such cosmetics easily fall off. Further, problems have also been posed recently in terms of safety and stability in that inorganic pigments have a strong surface activity and for this reason, accelerate oxidation of oily components used in cosmetics, whereby peroxides are formed to cause iritation to the skin, or perfumes are degenerated, etc. In order to solve such problems, various attempts to compound inorganic pigments which has undergone surface treatment have been heretofore made. However, cosmetics satisfying the aforesaid properties have not been realised yet. For example, improvement in adherence to the skin or in oil adsorptivity is attempted by treating the surface of inorganic pigments with silk fibroin (Japanese Published Examined Patent Application 11577/82). However, silk fibroin is not preferred in terms of safety and stability since it tends to easily putrify thereby to adversely affect the appearance and fragrance of cosmetics sometimes. Alternatively, there is known a method for preventing the surface activity of titanium dioxide by treating the surface with higher alcohol mono-esters of long chain aliphatic acids to render the surface hydrophobic (Japanese published Examined Patent Application 42167/83); however, titanium dioxide surface-treated with such esters is oily and provides undesired texture in spreading over the skin and accordingly, the amount compounded in cosmetics is limited to about 2% and its use is seriously restricted. In general, a method for rendering the surface hydrophobic by treating the surface of inorganic pigments with silicone oil of polysiloxanes is adopted. However, silicone oil often has a peculiar smell. Further the thus treated inorganic pigments are oily and poor in lubrication. For these reasons, cosmetics using them have an undesired texture in spreading over the skin.

The present inventors have found that long chain-acylated lysines are useful for improving the surface quality of inorganic materials and improve the dispersibility of inorganic fillers in resins, paints or organic solvents as described for the second preferred aspect of the invention. The present inventors have also found that when the inorganic pigments used therein are treated with the aforesaid long chain-acylated lysines, the inorganic pigments treated particularly with N-mono-acylated lysines with long chain-acyl provide not only improved dispersibility in and compatibility with oily raw materials but also greatly improved properties required for cosmetic compositions such as adherence to the skin, safety, stability, texture, etc. Further similar effects can be obtained by the cosmetics having compounded therein inorganic pigments treated with mono-acylated lysines containing long chain-acyl groups. It has been found that these effects are characteristics that are not observed with cosmetic compositions having compounded therein non-treated inorganic pigments of inorganic pigments surface-treated by conventional, known techniques and, the present invention has been accomplished. That is, the fourth preferred aspect of the present invention relates to cosmetic compositions having compounded therein inorganic pigments surface-treated

with N-mono-acylated lysines with acyl having a long chain. Examples of inorganic pigments used in this aspect of the present invention include talc, kaolin, titanium dioxide, titanium-coated mica, mica, iron oxides, silicic acid, ultramarine, Prussian blue, zinc flowers, clay, precipitated calcium carbonate, etc. The shape and size of these pigment particles are not particularly limited. The lysine may be optically active or in a racemic form. The long-chain acyl groups may be saturated or unsaturated, straight or branched aliphatic acyl groups having 8 to 22 carbon atoms, which may be a single chain or a mixed chain. Specific examples include 2-ethylhexanoyl, capryloyl, caproyl, lauroyl, myristoyl, palmitoyl, stearoyl, isostearoyl, oleoyl, behenoyl, cocyl, acyl derived from tallow fatty acids, acyl derived from hardened tallow fatty acids, etc. The site of the long chain acyl groups to be bound to the lysine is at the $\alpha$- or $\epsilon$-position of the amino groups. Specific examples include $N^{\epsilon}$-2-ethylhexanoyl-lysine, $N^{\epsilon}$-lauroyl-lysine, $N^{\epsilon}$-cocoyl-lysine, $N^{\epsilon}$-palmitoyl-lysine, $N^{\epsilon}$-isostearoyl-lysine, $N^{\epsilon}$-hardened tallow fatty acid-acyl-lysine, $N^{\alpha}$-capryloyl-lysine, $N^{\alpha}$-lauroyl-lysine, $N^{\alpha}$-myristoyl-lysine, $N^{\alpha}$-oleoyl-lysine, $N^{\alpha}$-behenoyl-lysine, etc. but are not limited thereto, of course.

In treating the inorganic pigments, it is preferred that the ratio of the N-mono-acylated lysine containing an acyl group having a long chain to the inorganic pigment be in a range of 0.05 to 5 wt%. With less than 0.05 wt%, the surface treatment effect is insufficient and, even though the ratio exceeds 5 wt%, no advantage is obtained from an economical viewpoint since the treatment effect is not so improved. Further depending upon necessity, other surface treatments may also be combined. As the methods of treating the pigments, any dry procedure or any wet procedure can be adopted. The dry procedure is simple and effective; the surface of the inorganic pigments can easily be treated either by mixing finely divided N-mono-acylated lysine powders with acyl having a long chain, with the inorganic pigments with stirring or by mixing the N-mono-acylated lysine with acyl having a long chain with the inorganic pigments and then co-pulverizing the mixture. Alternatively, the wet procedure can also be adopted in order to obtain the treatment effect with a smaller amount of the N-mono-acylated lysine with acyl having a long chain. In this case, the N-mono-acylated lysine with acyl having a long chain are hardly soluble in water which is approximately neutral or in oily raw materials conventionally used for cosmetics and accordingly, the quality of the surface of the inorganic pigments can be effectively modified by dissolving the N-mono-acylated lysine with acyl having a long chain in organic solvents using calcium chloride as a solubilizing agent, bringing the inorganic pigment into contact with the solution and then washing the mixture with water to remove calcium chloride. Alternatively, the surface treatment effect similar thereto can also be obtained by dissolving the N-mono-acylated lysine with long chain-acyl in alkaline water or in aqueous solvents, bringing the inorganic pigment into contact with the solution, neutralizing the mixture to render it about neutral, precipitating and adhering the N-mono-acylated lysine with long chain-acyl onto the surface of the pigment, washing the salt formed by neutralization with water to remove it and then drying.

The cosmetic composition having thus compounded therein the inorganic pigment thus surface-treated with the N-mono-acylated lysine with long chain-acyl possesses various advantages that are not observed with cosmetic compositions untreated or treated by conventionally known techniques. That is, (1) due to excellent dispersibility and compatibility of the inorganic pigment in oily raw materials, the products are homogeneous but not sandy and have a smooth texture; (2) the adherence to the skin is excellent so that makeup comes off only with difficulty; (3) the surface activity of the inorganic pigment is retarded so that peroxides are produced to a lesser extent, irritation caused by the peroxides to the skin is prevented and safety is excellent; (4) the quality of fragrance changes less and the stability of the product is excellent; (5) due to antibacterial activity of the N-mono-acylated lysine with long chain-acyl, contamination by microorganisms occurs only with difficulty, unlike silk fibroin; (6) cosmetic compositions having compounded therein silicone oil-treated inorganic pigments are stickly and have the disadvantage that spreading over the skin is poor but the cosmetic composition of the present invention has a dry texture and good spreading over the skin. As such, the cosmetic composition of the fourth aspect of the present invention overcomes the problems that have not been solved heretofore and is extremely desired.

This invention will now be illustrated by the following Examples.

Example 1:

In a solvent, a stated amount of $N^{\epsilon}$-lauroyl lysine and 1 g of an inorganic filler were shaken at 25°C for two hours in a solvent (1) or (2) as given in Table 1. The decrease of the amount of $N^{\epsilon}$-lauroyl lysine in the solvent due to the shaking treatment was determined by the absorbance at 210 nm and reported in Table 1 as the amount of the compound adsorbed per g of the inorganic filler.

It is noted from Table 1 that the amount of adsorption of $N^{\epsilon}$-lauroyl lysine on various inorganic fillers substantially reached the saturation value at an extremely low application rate of about 12.50 mg. It is also noted that addition of a small amount of water, a non-solvent, promoted the adsorption and enabled further reduction of the application rate.

The inorganic fillers treated by the method described above invariably showed a critical surface tension of about 30 dynes/cm, indicating that the surface affinity for water was sufficiently repressed.

To these inorganic fillers, the $N^{\epsilon}$- lauroyl lysine exhibited very high adsorptive strength. The critical surface tensions showed no discernible change after the inorganic fillers were boiled in toluene for 10 minutes.

Examples 2A to 2I

In a container holding 25 ml of an organic solvent as given in the following Tables 2-5, 0.05 g of a surface modifier and 0.5 g of an inorganic filler were shaken on an ultrasonic shaker for 10 minutes. The container was then left standing in an erect position and placed under visual observation to rate the condition of dispersion of the inorganic filler. The rating was made on a three-point scale, wherein the circle o denotes uniform dispersion, the triangle Δ partial coagulation and separation, and the cross x thorough separation.

It is noted from the results of the tests given in Tables 2-5 that the surface modifiers of this invention manifested excellent dispersing properties in various organic solvents and showed no discrimination in respect of the choice of inorganic filler.

TABLE 1 Adsorption of the surface modifier to the inorganic materials(mg/g)

| Inorganic materials | Solvent | Volume of $N^{\varepsilon}$-lauroyl-L-lysine (mg) | | | | | |
|---|---|---|---|---|---|---|---|
| | | 6.25 | 12.50 | 18.75 | 25.00 | 31.25 | 37.50 |
| $CaCO_3$ | (1) | 1.5 | 2.7 | 3.2 | 3.4 | 3.5 | 3.5 |
| | (2) | 2.0 | 3.9 | 4.5 | 4.8 | 4.8 | 5.1 |
| $\alpha-Fe_2O_3$ | (1) | 3.6 | 4.5 | 4.5 | 4.5 | 3.5 | 3.1 |
| | (2) | – | – | – | – | – | – |
| $TiO_2$ | (1) | 2.7 | 3.3 | 3.6 | 4.3 | 4.6 | 5.0 |
| | (2) | 4.6 | 7.3 | 8.0 | 8.6 | 9.6 | 10.6 |

Solvent (1) Ethanol ...... 25.00 g
$CaCl_2$ ....... 0.50 g

Solvent (2) Ethanol ...... 23.75g
Water....... 1.25g
$CaCl_2$ ....... 0.50g

TABLE 2 Dispersion of the inorganic materials in dichloroethane

| Surface modifier | Inorganic materials | | | |
|---|---|---|---|---|
| | $CaCO_3$ | $Mg(OH)_2$ | $SiO_2$ | $\gamma-Fe_2O_3$ |
| Untreated | × | × | × | × |
| stearic acid | ○ | △ | △ | △ |
| AOT* | ○ | ○ | ○ | × |
| Example (1) | ○ | ○ | ○ | ○ |
| (2A) | ○ | ○ | ○ | ○ |
| (2B) | ○ | ○ | ○ | ○ |

TABLE 3 Dispersion of the inorganic materials in n-decane

| Surface modifier | Inorganic materials | | | |
|---|---|---|---|---|
| | $CaCO_3$ | $Mg(OH)_2$ | $SiO_2$ | $\gamma-Fe_2O_3$ |
| untreated | × | × | × | × |
| stearic acid | △ | ○ | × | × |
| A O T* | ○ | △ | △ | △ |
| Example (2C) | ○ | ○ | ○ | ○ |
| (2D) | ○ | ○ | △ | ○ |

TABLE 4 Dispersion of the inorganic materials in DBP

| Surface modifier | Inorganic materials | | | |
|---|---|---|---|---|
| | $CaCO_3$ | $Mg(OH)_2$ | $SiO_2$ | $\gamma-Fe_2O_3$ |
| untreated | ○ | × | ○ | ○ |
| stearic acid | ○ | △ | ○ | ○ |
| A O T* | ○ | ○ | × | × |
| Example (2E) | ○ | ○ | △ | ○ |
| (2F) | △ | ○ | ○ | ○ |

TABLE 5 Dispersion of the inorganic materials in toluene

| Surface modifier | Inorganic materials | | | |
|---|---|---|---|---|
| | $CaCO_3$ | $Mg(OH)_2$ | $SiO_2$ | $\gamma-Fe_2O_3$ |
| untreated | X | X | X | X |
| stearic acid | X | X | X | X |
| A O T* | O | O | O | O |
| Example (2G) | O | O | O | O |
| (2H) | O | O | O | O |
| (2I) | O | O | O | O |

Example

- (1) $N^{\alpha}$- Lauroyl-L-lysine
- (2A) $N^{\varepsilon}$-Stearoyl-L-lysine
- (2B) $N^{\alpha}$-$N^{\varepsilon}$-Di-lauroyl-L-lysine
- (2C) $N^{\alpha}$-Oreoyl-L-lysine
- (2D) $N^{\alpha}$-Cocoyl-L-lysine
- (2E) $N^{\varepsilon}$-Stearoyl-L-lysine
- (2F) $N^{\alpha}$-Stearoyl-$N^{\varepsilon}$-lauroyl-L-lysine
- (2G) (2A): Stearic acid = 1:1 (mix.)
- (2H) $N^{\varepsilon}$-Stearoyl-L-lysine
- (2I) $N^{\alpha}$-Cocoyl-L-lysine
- AOT : Sodium di(2-ethylhexyl) sulphosuccinate obtained from KAO Atlas Co Ltd, Japan

Example 3 (Face Powder)

| | |
|---|---|
| $N^{\alpha}$-octanoyl-L-lysine | 35.0 wt% |
| $N^{\varepsilon}$-palmitoyl-L-lysine | 30.0 |
| $N^{\alpha}$-$N^{\varepsilon}$-dilauroyl-L-lysine | 35.0 |
| Perfume | any suitable amount |

A face powder having the above composition is prepared by-mixing in a ball mill. The product was very mild and safe for the human skin, had the properties of good spreading over the skin and good adhesion to the skin.

Example 4 (Face Powder)

| | |
|---|---|
| $N^{\varepsilon}$-lauroyl-L-lysine | 40.0 wt% |
| Talc | 43.0 |
| Kaolin | 10.0 |
| $CaCO_3$ (precipitated) | 2.0 |
| ZnO | 2.0 |
| Zn-stearate | 2.0 |
| $CaCO_3$ | 1.0 |
| Pigment | any suitable amount |
| Aromatics | any suitable amount |

A face powder having the above composition is prepared by mixing in a ball mill. The product was very mild and safe for the human skin, had good properties of adhesion to the skin and good adsorption of secretion (sweat).

Example 5 (Face paste)

| | |
|---|---|
| $N^{\varepsilon}$-cocoyl-L-lysine (more than 74 micrometres (200 mesh)) | 40.0 wt% |
| Nylon 12 | 20.0 |
| Glycerin | 10.0 |
| Sodium DL-pyrrolidone carboxylate | 3.0 |
| Pure Water | 27.0 |
| Pigment | any suitable amount |

A face paste having the above composition is prepared by mixing in a ball mill. The produce had good properties of spreading over the skin and adhesion to the skin, and had good toilet maintenance properties.

Example 6 (Solid Face Powder)

| | |
|---|---|
| $N^{\alpha}$-stearoyl-L-lysine | 20.0 wt% |
| $N^{\varepsilon}$-stearoyl-L-lysine | 20.0 |
| $N^{\alpha}$-$N^{\varepsilon}$-distearoyl-L-lysine | 10.0 |
| Talc | 10.0 |
| ZnO | 10.0 |
| Kaolin | 10.0 |
| Zn-stearate | 5.0 |
| $MgCO_3$ | 5.0 |
| Tragant gum aqueous soln.(as binder) | 7.0 |
| Sodium DL-pyrrolidone carboxylate | 3.0 |
| Pigment, perfume | any suitable amounts |

A solid face powder having the above composition is prepared by press-moulding after mixing the above materials. The product has a comfortable and soft feel to the skin.

Example 7 (Eye Shadow)

| | |
|---|---|
| $N^{\varepsilon}$-stearoyl-L-lysine (more than 74 micrometres (200 mesh)) | 8.0 wt% |
| Talc | 1.0 |
| Pigment | 12.0 |
| Peal pigment | 18.0 |
| White Vaseline | 12.0 |

An eye shadow having the above composition is prepared by mixing in an emulsifier. The product had good properties of spreading over the skin and adhesion to the skin, and had good toilet maintenance properties.

Example 8

In 100 g of a methanol 1% lysine laurate solution, 100 g of titanium dioxide was stirred for 10 minutes. The resultant solution was heated under a vacuum to expel methanol and cause the surface of titanium dioxide to adsorb lysine laurate. The composite thus obtained was heated at 160°C for two hours under an atmosphere of nitrogen to produce modified titanium dioxide. Then the product on the surface of this modified titanium dioxide was extracted with 100 ml of acetic acid and analyzed by HPLC. By comparison with standard substances, it was identified to be N-lauroyl-L-lysine.

Conditions of analysis

| | |
|---|---|
| Column: | YMC-PACK A-311, 100 mm x 6mm Diam. (Yamamura Chemical Laboratory) |
| Eluent: | 0.1M $NaH_2PO_4$/MeOH (2/8) |
| Wavelength: | 210 nm |
| Flow volume: | 1.0 ml/min |

Example 9 (Dispersibility of titanium dioxide)

A) Preparation of sample

In 100 g of a methanol 1% lysine laurate solution, 100 g of titanium dioxide (Sample 1) was stirred for 10 minutes. The resulting solution was heated under a vacuum to expel methanol and cause the surface of titanium dioxide to adsorb lysine laurate (Sample 2). In an atmosphere of nitrogen, Sample 2 was heated at 160°C for two hours to prepare modified titanium dioxide (Sample 3). Then, Sample 2 and Sample 3 were each stirred and washed in a 1:1 water/methanol solution (60°C) 100 times as large in volume as each sample and then dried under a vacuum, to produce Samples 4, 5 respectively.

B) Method of determination and results

Each of the samples, Samples 1-5, was added to toluene in a concentration of 0.2% and exposed to ultrasonic waves for stirring and dispersion. The resultant dispersion was placed in a cell and subjected to an acceleration 3 times the gravitational acceleration to induce forced precipitation. The stability of dispersion was found based on the change of the adsorbance of the dispersion with time. The results are shown in Figs 1 to 5 respectively.

It is noted from the results of Figs. 1 to 5 that the titanium dioxide treated by the method of surface modification of the present invention acquired notably enhanced dispersibility in toluene and, even after washing with the water-methanol solution at 60°C, retained this effect intact. Thus, it was shown to possess outstanding resistance to water and solvents.

Example 10 (Dispersibility of Kaolin)

A) Preparation of sample

In 100 g of a methanol 1% lysine palmitate solution, 100 g of kaolin (Sample 6) was stirred for 10 minutes. The resultant solution was heated under a vacuum to expel methanol and cause the surface of kaolin to adsorb lysine palmitate (Sample 7). In an atmosphere of nitrogen, Sample 7 was heated at 160°C for two hours to prepare modified kaolin (Sample 8). Then, Samples 7 and 8 were each stirred and washed in a 1:1 water/methanol solution (60°C) 100 times as much in volume as the sample and dried under a vacuum to obtain Samples 9 and 10 respectively.

B) Method of determination and results

Each of Samples 6-10 was added in a concentration of 0.2% to liquid paraffin and mixed and dispersed by exposure to ultrasonic waves. The dispersion was placed in a cell and subjected to an acceleration 3 times the gravitational acceleration to induce forced precipitation. The stability dispersion was determined based on the change of the absorbance with time. The results are shown in Figs. 6 to 10 respectively.

It is noted from the results of Figs. 6 to 10 that the kaolin treated by the method of surface modification of the present invention acquired notably enhanced dispersibility in liquid paraffin and, even after washing in the water-methanol solution at 60°C. retained this effect intact. Thus, it was shown to possess outstanding resistance to water and solvents.

Example 11 (Dispersibility of talc)

A) Preparation of sample

In 100 g of a methanol solution of 1% ornithine salt of palm oil fatty acid, 100 g of talc (Sample 11) was stirred for 10 minutes. The resultant solution was heated under a vacuum to expel methanol and cause the surface of kaolin to adsorb the ornithine salt of palm oil fatty acid (Sample 12). In an atmosphere of nitrogen gas, Sample 12 was heated at 160°C for two hours to prepare improved talc (Sample 13). Then, Samples 12 and 13 were each stirred and washed in a 1:1 water-methanol solution (60°C) 100 times as much in volume as the sample and dried under a vacuum to produce Samples 14 and 15 respectively.

B) Method of determination and results

Each of the samples was added in a concentration of 0.2% to liquid paraffin and mixed and dispersed by exposure to ultrasonic waves. This dispersion was placed in a cell and subjected to an acceleration 3 times the gravitational acceleration to induce forced precipitation. The stability of dispersion was determined based on the change of the adsorbancy with time. The results are shown in Fig. 11 to Fig. 15 respectively.

It is noted from the results of Fig. 11 to Fig. 15 that the talc treated by the method of surface modification of the present invention acquired notably enhanced dispersibility in liquid paraffin and, even after washing in the water/methanol solution at 60°C, retained its effect intact. Thus, it was shown to possess outstanding resistance to water and solvents.

Each figure shows the dispersive power of each sample in the solvent.

Sample 1-5 in example 9 in toluene is shown in Fig. 1-5, sample 6-10 in example 10 in liquid paraffin is shown in 6-10, sample 11-15 in example 11 in liquid paraffin is shown in Fig. 11-15.

Example 12

Each of the samples of Examples 9 to 11 was placed in the amount of 0.5 g in a container holding 25 ml of an organic solvent indicated in the following Tables 6 to 8. The container was shaken on an ultrasonic shaker for 10 minutes. It was then left standing at rest in an erect position and kept under visual observation to rate the condition of dispersion of the sample.

The Experimental results as to the dispersive power of the inorganic powder whose surface is treated with the surface modifiers are shown in Tables 6 to 8.

The results indicate that the treated inorganic powder forms good dispersions in organic solvents.

Table-6 Dispersion in dichloroethane

| | | | | |
|---|---|---|---|---|
| Sample | 1 | in example | 9 | : not good |
| " | 6 | " | 10 | : not good |
| " | 11 | " | 11 | : not good |
| " | 3 | " | 9 | : good |
| " | 8 | " | 10 | : good |
| " | 13 | " | 11 | : good |

Table-7 Dispersion in n-decane

| | | | | |
|---|---|---|---|---|
| Sample | 1 | in example | 9 | : not good |
| " | 6 | " | 10 | : not good |
| " | 11 | " | 11 | : not good |
| " | 3 | " | 9 | : good |
| " | 8 | " | 10 | : good |
| " | 13 | " | 11 | : good |

Table-8 Dispersion in dibutyl phthalate

| | | | | |
|---|---|---|---|---|
| Sample | 1 | in example | 9 | : not good |
| " | 6 | " | 10 | : not good |
| " | 11 | " | 11 | : not good |
| " | 3 | " | 9 | : good |
| " | 8 | " | 10 | : good |
| " | 13 | " | 11 | : good |

Example 13 (Face Powder)

| | | | |
|---|---|---|---|
| Sample 3 | in example | 9 | 35.0 wt % |
| Sample 8 | " | 10 | 30.0 |
| Sample 13 | " | 11 | 35.0 |
| Pigment | | | any suitable amount |
| Perfume | | | any suitable amount |

The surface modified inorganic powder, samples 3, 8 and 13, was prepared by washing it with methanol and drying it. The face powder, having the above composition is prepared by mixing in a ball mill. The produce was very mild and safe for the human skin, had the properties of good spreading over the skin and good adhesion to the skin.

Example 14 (Face Paste)

| | | | |
|---|---|---|---|
| Sample 3 in example 9 (more than 74 micrometres (200 mesh)) | | | 10.0wt% |
| Sample 8 | " | 10 | 15.0 |
| Sample 13 | " | 11 | 35.0 |
| Glycerin | | | 10.0 |
| Sodium DL-pyrrolidone carboxylate | | | 3.0 |
| Liquid paraffin | | | 27.0 |
| Pigment and perfume | | | any suitable amounts |

The surface modified inorganic powder, sample 3, 8 and 13, was prepared by washing it with methanol and drying it. A face paste having the above composition is prepared by mixing in an emulsifier apparatus. The product had the good properties of spreading over the skin and adhesion to the skin, and had the ability to maintenance the toilet.

Example 15 (Solid Face Powder)

| | | | |
|---|---|---|---|
| Sample 3 in example 9 (more than 74 micrometres (200 mesh)) | | | 20.0wt% |
| " 8 | " | 10 | 30.0 |
| " 13 | " | 11 | 30.0 |
| Zn-stearate | | | 5.0 |
| $MgCO_3$ | | | 5.0 |
| Tragant gum aqueous soln. | | | 7.0 |
| DL-pyrrolidone carboxylate | | | 3.0 |
| Pigment | | | any suitable amount |
| Perfume | | | " " " |

The surface modified inorganic powder, samples 3, 8 and 13, was prepared by washing it with methanol and drying it. A solid face powder having above composition is prepared by press-moulding after mixing the above materials. The product has a comfortable and soft feel to the skin.

Example 16 (Eye Shadow)

| | |
|---|---|
| Sample 3 in example 9 (more than 74 micrometres (200 mesh)) | 9.0wt% |
| Pigment | 12.0 |
| Peal agent | 18.0 |
| White Vaseline | 12.0 |
| Lanolin derivative | 5.0 |
| Micro crystalline wax | 15.0 |
| Squalane | 2.0 |
| Liquid paraffin | 27.0 |

The surface modified inorganic powder, sample 3, was prepared by washing it with methanol and drying it. An eye-shadow having above composition is prepared by press-moulding after mixing the above materials in an emulsifier apparatus. The product provides a comfortable and soft feel to the skin.

Example 17

The peroxide values of olive oil treated by samples 1, 2 and 3 of Example 9 are shown below. 25 ml of olive oil solutions in which are dispersed samples 1, 2 and 3, each in an amount of 0.025 g, were heated at 98°C in an air stream. After 30 hours of heating, the peroxide value of the olive oil was detected.

| | | | | The peroxide value (POV) |
|---|---|---|---|---|
| Sample | 1 | in example | 9 | 160 |
| " | 2 | " | 9 | 45 |
| " | 3 | " | 9 | 7 |

It is clear from the results of this experiment that titanium dioxide treated with this surface modifier method was suppressed the active power of the surface of it, therefore did not accelerate the decomposition of oil surrounding it and the unsafe peroxide to the skin scarcely occurred in olive oil. We conclude from the experiments described above that the modifiers and surface modified inorganic materials was safe and had the property to suppress the decomposition of base oil surrounding them.

Example 18 (Eye Shadow)

| | | | |
|---|---|---|---|
| A: | Chromium oxide | 20 | parts by weight |
| | Mica coated with bismuth oxychloride | 20 | " |
| | Talc | 25 | " |
| | Sericite | 15 | " |
| B: | Squalane | 10 | " |
| | Lanoline | 7 | " |

To Component A, 3 parts by weight of a mixture of $N^{\epsilon}$-palmitoyl-L-lysine and $N^{\alpha}$-cocoyl-L-arginine (weight ratio 1:1) having an average particle size of 2 micrometres were added and the mixture was agitated for 10 minutes with a Hensil mixer to effect the surface treatment.

To the surface-treated Component A, Component B was added. After agitating and mixing the mixture with a Hensil mixer, the inner temperature of which was kept at 60°C, for 15 minutes, the mixture was cooled to 40°C and withdrawn. The mixture was moulded to provide the product. The product had homogeneous compatibility of the inorganic pigments in Component A with Component B and smooth texture without being sandy, upon applicaiton.

Next, 1 g of this product was spread over a Petri dish having a diameter of 4 cm, which was exposed to a xenon lamp (Xenon Tester XF-60S, made by Shimazu) for 2 hours from a distance of 1 m. The insoluble matter was dissolved out with n-hexane and the amount of peroxides produced in the solute was determined. The peroxide value of the product after exposure to the xenon lamp was 6.5. For purpose of comparison, Component A was treated with 3 parts by weight of magnesium stearate and an eye shadow was prepared thereafter in a similar fashion, which eye shadow was exposed to a xenon lamp under the same conditions and the amount of peroxides produced was determined. The peroxide value after exposure was 17.7. As such, the formation of peroxides is prevented with the eye shadow of the present invention and its safety is excellent.

Example 19 (Face Powder)

In 2500 g of ethanol, 50 g of calcium chloride was dissolved and 2.5 g of $N^{\epsilon}$-lauroyl-L-lysine was dissolved in the solution. To the solution, a mixture of 80 g of talc and 5 g of zinc flower was added and the mixture was stirred for 2 hours at room temperature. The mixture of talc and zinc flower was removed by filtration. After thoroughly washing with water to remove calcium chloride, drying was performed at 80°C overnight under reduced pressure. $N^{\epsilon}$-lauroyl-L-lysine remained in the filtrate and the washing liquid was quantitatively assayed by liquid chromatography. As a result, the amount of N-lauroyl-L-lysine adsorbed on the mixture of talc and zinc flower was 0.35 g. Using the thus treated talc and zinc flower, a powder having the following composition was prepared.

| | |
|---|---|
| A. Talc treated with $N^{\epsilon}$-lauroyl-L-lysine | 80 parts by weight |
| B. Zinc flower treated with $N^{\epsilon}$-lauroyl-L-lysine | 5 " |
| Potato starch | 5 " |
| Zinc stearate | 3 " |
| Perfume | 0.1 " |

For purpose of comparison, powders having the above composition were prepared except that untreated talc and untreated zinc flower were used instead of A and B, respectively. The powders were evaluated by 15 females aged 20 to 40 years. The following results were obtained:

| | Prefer the powder of this invention | Prefer the powder for comparison | Hard to say which |
|---|---|---|---|
| Adherence under ordinary condition | 9 | 2 | 4 |
| Adherence in sweating | 12 | 0 | 3 |
| (Numerals indicated the number of females who gave reply) | | | |

From the results described above, it will be understood that the powder of the present invention is excellent in adherence to the skin and has good adherence particularly in sweating.

Example 20 (Solid Foundation)

| | |
|---|---|
| A. Iron oxide yellow | 0.8 part by weight |
| Indian red | 0.5 " |
| Titanium oxide | 11.0 " |
| Zinc oxide | 8.5 " |
| Talc | 25.0 " |
| Kaolin | 41.5 " |
| B. $N^{\alpha}$-Cocoylorinithine (ave.particle size 2 micrometres) | 1.0 " |
| $N^{\alpha}$-Stearoylhistidine ( " " " 3 micrometres) | 2.5 " |
| C. Liquid paraffin | 6.0 " |
| 1,3-Butyleneglycol | 3.0 " |
| Perfume | 0.2 " |

Component A was mixed for 10 minutes using a Hensil mixer and Component B was added thereto followed by stirring and mixing for further 10 minutes, whereby the surface treatment was effected.

Component C was added to the surface-treated Component A. After mixing and stirring the mixture for 20 minutes, the perfume was were added thereto at temperatures lower than 35°C. Stirring was performed for further 10 minutes. After withdrawing the mixture, it was subjected to press moulding to prepare a solid foundation. The product was homogeneous and non-sticky and had smooth texture; when applied to the skin, spreading over the skin was good and adherence to the skin was good.

## Claims

**1.** An inorganic substance whose surface has been modified with one or more N-mono- or N,N'-di-acylated lysines, wherein the acyl group in the N-mono-acylated lysine is an aliphatic acyl group having from 8 to 22 carbon atoms, and each of the acyl groups, which may be the same or different, in the N,N'-di-acylated lysine is an aliphatic acyl group having from 1 to 22 carbon atoms with the proviso that the two aliphatic acyl groups together have from 9 to 44 carbon atoms.

**2.** A substance according to Claim 1, wherein the N-mono- and N,N'-di-acylated lysines are chosen from those having the following general formulae:

$$RCONH(CH_2)_4-\underset{\displaystyle NH_2}{\underset{|}{CH}}-COOH \qquad (1)$$

$$H_2N(CH_2)_4-\underset{\displaystyle NHCOR}{\underset{|}{CH}}-COOH \qquad (2)$$

$$R'CONH(CH_2)_4-\underset{\displaystyle NHCOR''}{\underset{|}{CH}}-COOH \qquad (3)$$

wherein the RCO group in general formulae (1) and (2) represents an aliphatic acyl group having from 8 to 22 carbon atoms; and R'CO and R''CO in general formulae (3) each represent an aliphatic acyl group having from 1 to 22 carbon atoms with the proviso that the two aliphatic acyl groups together have from 9 to 44 carbon atoms.

**3.** A substance according to Claim 1, wherein the N-mono-acylated lysine is an $N^{\alpha}$-long chain acyl-lysine or an $N^{\epsilon}$-long chain acyl-lysine.

**4.** A substance according to any of Claims 1 to 3, being in powder form.

**5.** A cosmetic product comprising a substance as claimed in Claim 4.

**6.** A process for the modification of the surface of an inorganic substance, which process comprises causing the surface to adsorb a fatty acid salt of lysine, wherein the fatty acid has from 8 to 22 carbon atoms, and subsequently heating the surface of the inorganic substance to a temperature in the range of from 100 ° C to 250 ° C to covert the fatty acid salt of lysine into an N-mono- or N,N'di-acylated lysine as defined in Claim 1.

## Revendications

**1.** Substance minérale dont la surface a été modifiée avec une ou plusieurs lysines N-mono- ou N,N'-diacylées dont le groupe acyle dans la lysine N-monoacylée est un groupe acyle aliphatique ayant de 8 à 22 atomes de carbone et chacun des groupes acyles, qui peuvent être semblables ou différents, dans la lysine N,N'-diacylée, est un groupe acyle aliphatique ayant 1 à 22 atomes de carbone, sous réserve que les deux groupes acyles aliphatiques aient ensemble de 9 à 44 atomes de carbone.

**2.** Substance selon la revendication 1, dans laquelle les lysines N-mono- et N,N'-diacylées sont choisies parmi celles répondant aux formules générales suivantes :

$$RCONH(CH_2)_4-\underset{NH_2}{\underset{|}{CH}}-COOH \qquad (1)$$

$$H_2N(CH_2)_4-\underset{NHCOR}{\underset{|}{CH}}-COOH \qquad (2)$$

$$R'CONH(CH_2)_4-\underset{NHCOR''}{\underset{|}{CH}}-COOH \qquad (3)$$

où le groupe RCO, dans les formules générales (1) et (2), représente un groupe acyle aliphatique ayant de 8 à 22 atomes de carbone ; et R'CO et R''CO, dans les formules générales (3), représentent chacun un groupe acyle aliphatique ayant de 1 à 22 atomes de carbone, sous réserve que les deux groupes acyles aliphatiques ensemble aient de 9 à 44 atomes de carbone.

**3.** Substance selon la revendication 1, dans laquelle la lysine N-monoacylée est une $N^{\alpha}$-(acyl à chaîne longue)lysine ou une $N^{\epsilon}$-(acyl à chaîne longue)lysine.

**4.** Substance selon l'une quelconque des revendications 1 à 3 qui est sous forme d'une poudre.

**5.** Produit cosmétique comprenant une substance selon la revendication 4.

**6.** Procédé pour la modification de la surface d'une substance minérale, lequel procédé consiste à faire adsorber par la surface un sel d'acide gras de lysine, dont l'acide gras a 8 à 22 atomes de carbone, puis à chauffer la surface de la substance minérale à une température dans la gamme de 100 °C à 250 °C pour transformer le sel d'acide gras de lysine en une lysine N-mono- ou N,N'-diacylée, comme défini dans la revendication 1.

## Patentansprüche

**1.** Anorganische Substanz, deren Oberfläche mit einem oder mehreren N-mono- oder N,N'-di-acylierten Lysinen modifiziert worden ist, wobei die Acylgruppe in dem N-monoacylierten Lysin eine aliphatische Acylgruppe mit 8 bis 22 Kohlenstoffatomen ist und jede der Acylgruppen, die gleich oder verschieden sein können, in dem N,N'-di-acylierten Lysin eine aliphatische Acylgruppe mit 1 bis 22 Kohlenstoffatomen ist, mit der Maßgabe, daß die beiden aliphatischen Acylgruppen zusammen 9 bis 44 Kohlenstoffatome aufweisen.

2. Substanz nach Anspruch 1, in der das N-mono- und N,N'-di-acylierte Lysin unter Verbindungen der folgenden allgemeinen Formeln ausgewählt sind :

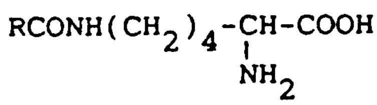 (1)

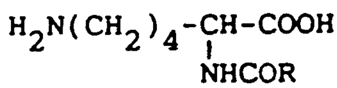 (2)

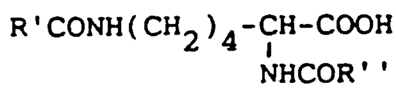 (3)

worin die RCO-Gruppe in den allgemeinen Formeln (1) und (2) eine aliphatische Acylgruppe mit 8 bis 22 Kohlenstoffatomen bedeutet, und R'CO und R''CO in der allgemeinen Formel (3) jeweils eine aliphatische Acylgruppe mit 1 bis 22 Kohlenstoffatomen bedeuten, mit der Maßgabe, daß die beiden aliphatischen Acylgruppen zusammen 9 bis 44 Kohlenstoffatome aufweisen.

3. Substanz nach Anspruch 1, wobei das N-mono-acylierte Lysin ein $N^{\alpha}$-langkettig-Acyl-Lysin oder ein $N^{\varepsilon}$-langkettig-Acyl-Lysin ist.

4. Substanz nach einem der Ansprüche 1 bis 3 in Pulverform.

5. Kosmetisches Produkt, enthaltend eine Substanz gemäß Anspruch 4.

6. Verfahren zur Modifizierung der Oberfläche einer anorganischen Substanz, welches umfaßt das Adsorbieren eines Fettsäuresalzes von Lysin an der Oberfläche, wobei die Fettsäure 8 bis 22 Kohlenstoffatome aufweist, und anschließendes Erhitzen der Oberfläche der anorganischen Substanz auf eine Temperatur im Bereich von 100 ° C bis 250 ° C, wobei das Fettsäuresalz von Lysin in ein N-mono- oder N,N'-di-acyliertes Lysin gemäß der Definition in Anspruch 1 umgewandelt wird.

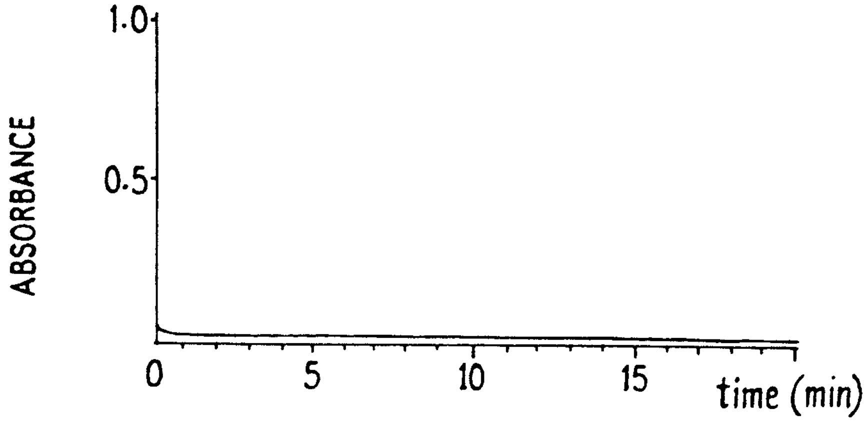


FIG.1 DISPERSION OF SAMPLE 1

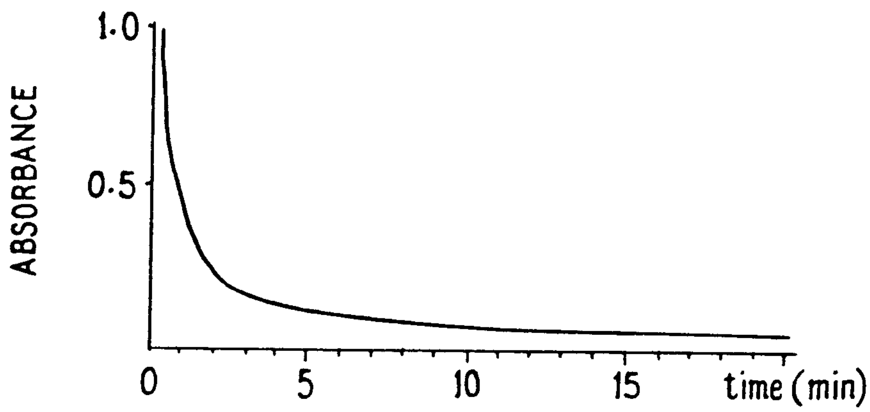


FIG.2 DISPERSION OF SAMPLE 2

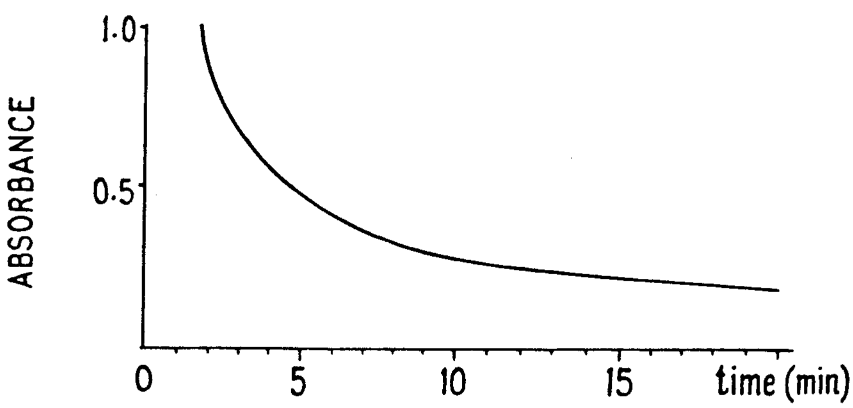


FIG. 3 DISPERSION OF SAMPLE 3

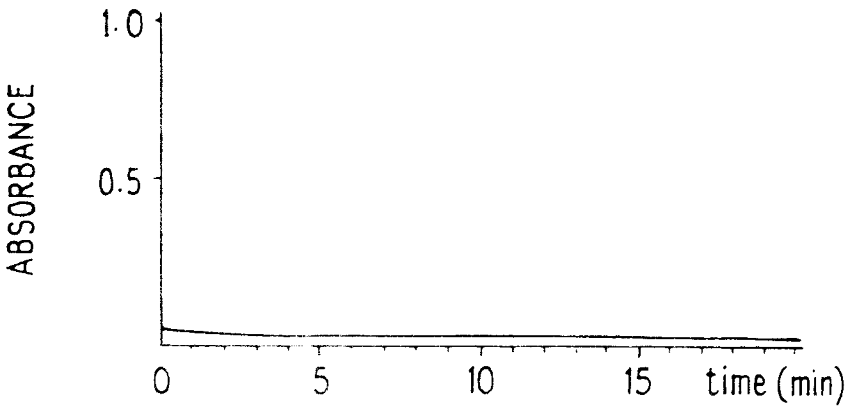


FIG.4 DISPERSION OF SAMPLE 4

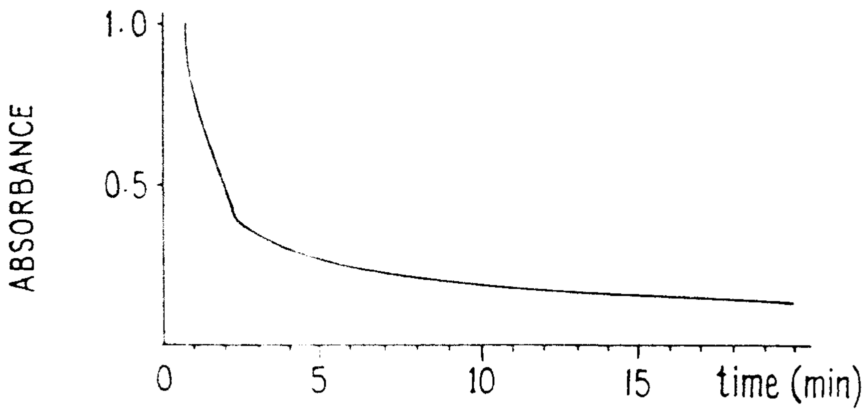


FIG.5 DISPERSION OF SAMPLE 5

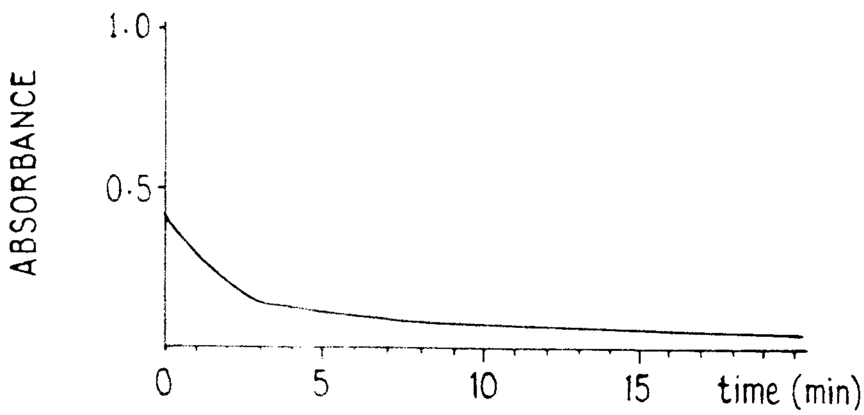


FIG.6 DISPERSION OF SAMPLE 6

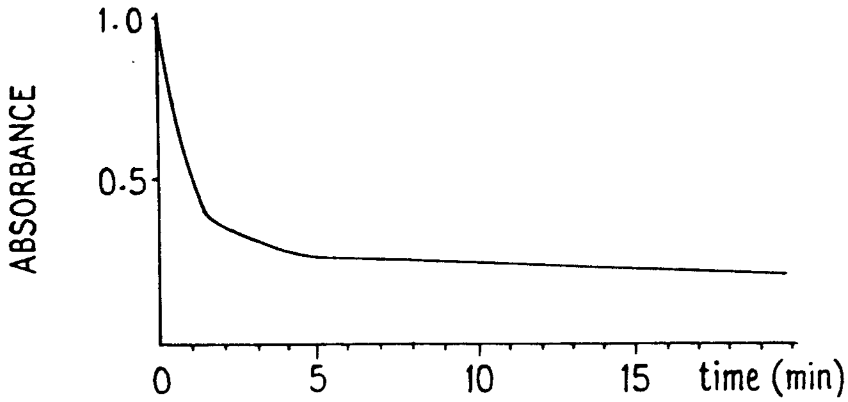


FIG.7 DISPERSION OF SAMPLE 7

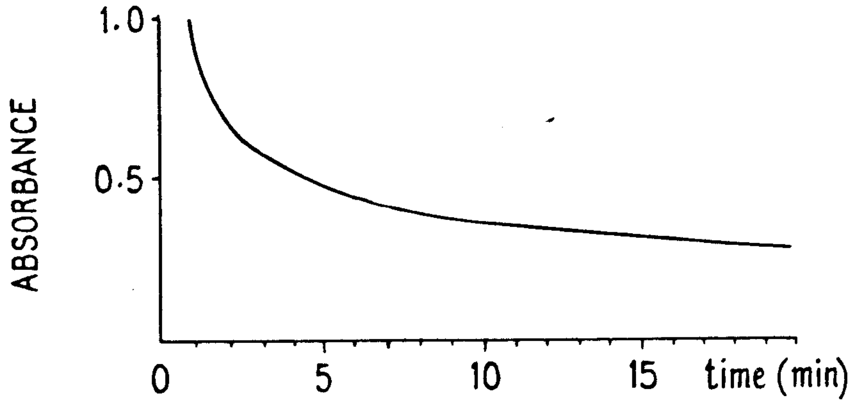


FIG.8 DISPERSION OF SAMPLE 8

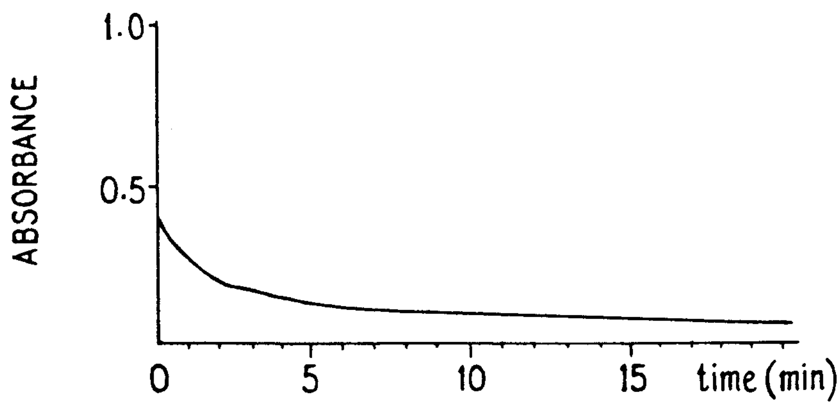


FIG.9 DISPERSION OF SAMPLE 9

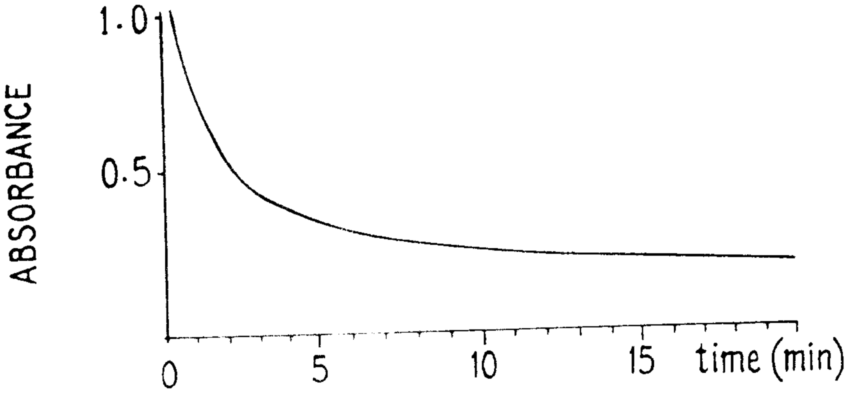


FIG.10 DISPERSION OF SAMPLE 10

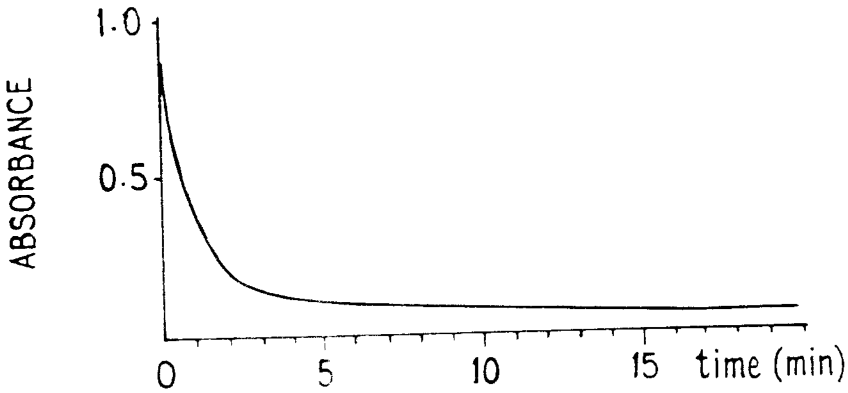


FIG.11 DISPERSION OF SAMPLE 11

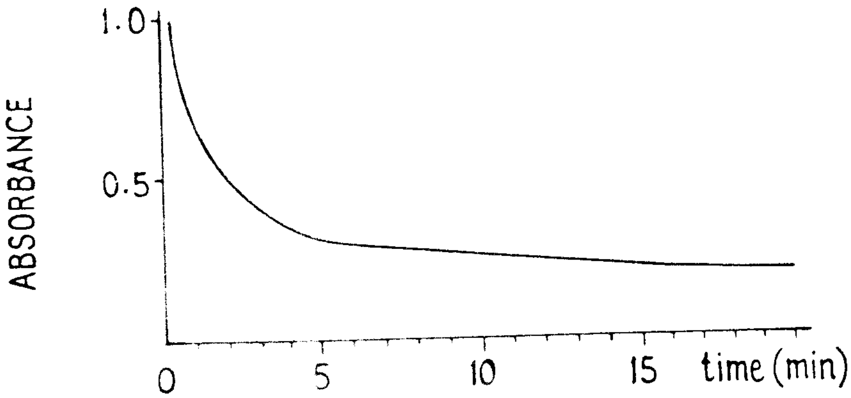


FIG.12 DISPERSION OF SAMPLE 12

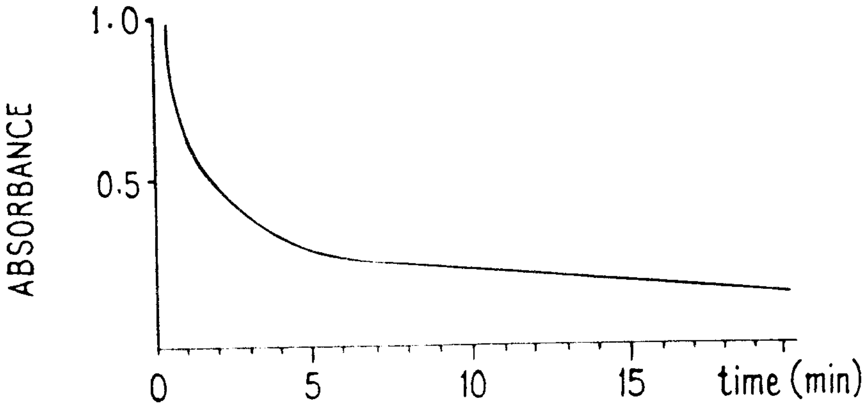


FIG. 13 DISPERSION OF SAMPLE 13

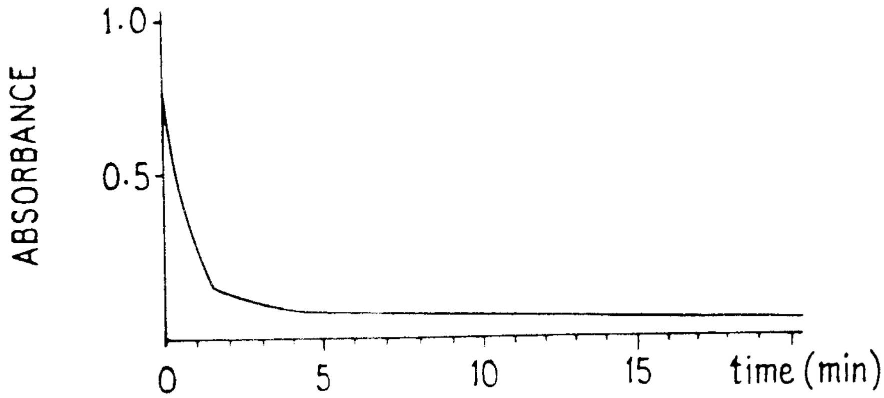


FIG. 14 DISPERSION OF SAMPLE 14

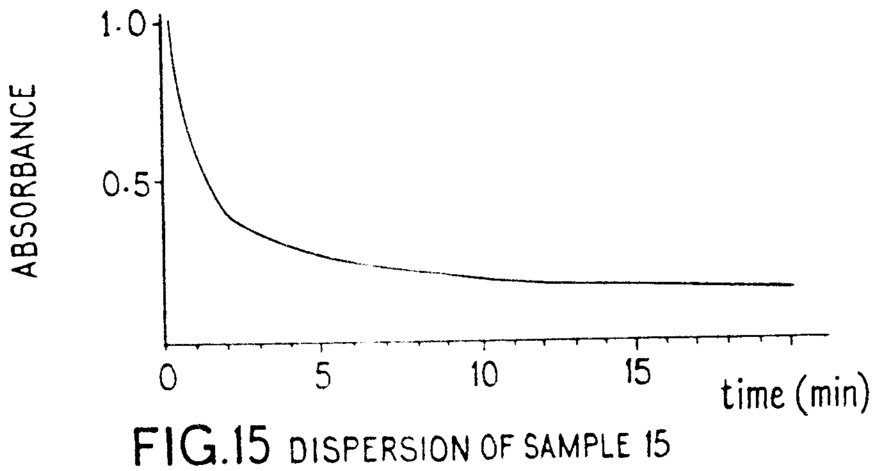


FIG. 15 DISPERSION OF SAMPLE 15